# EUROPEAN PATENT SPECIFICATION

(11) **EP 1 388 009 B1**
(45) Date of publication and mention of the grant of the patent: **28.06.2006**
(21) Application number: 02726250.0
(22) Date of filing: 10.05.2002
(51) Int. Cl.: G01N 33/53

(54) **SURF2LEAD**
SURF2LEAD
SURF2LEAD

(30) Priority: 11.05.2001 US 290070 P
(43) Date of publication of application: 11.02.2004
(73) Proprietor: The Genetics Company Inc., 8952 Schlieren (CH)
(72) Inventor: DIETRICH, Axel, 65929 Frankfurt (DE); ENGKVIST, Ola, Max-Lebsche-Platz 32, 81377 München (DE); RESTER, Ulrich, 64295 Darmstadt (DE); WANG, Li-hsing, 10707 Berlin (DE); WREDE, Paul, 14195 Berlin (DE)
(74) Representative: Dey, Michael
(86) International application number: PCT/EP2002/005180
(87) International publication number: WO 2002/092218

(56) References cited:
- KUNTZ I D ET AL: "A GEOMETRIC APPROACH TO MACROMOLECULE-LIGAND INTERACTIONS" JOURNAL OF MOLECULAR BIOLOGY, LONDON, GB, vol. 161, no. 2, 25 October 1982 (1982-10-25), pages 269-288, XP000560868 ISSN: 0022-2836
- KIRKPATRICK D L ET AL: "STRUCTURE-BASED DRUG DESIGN: COMBINATORIAL CHEMISTRY AND MOLECULAR MODELING" COMBINATORIAL CHEMISTRY AND HIGH THROUGHPUT SCREENING, HILVERSUM, NL, vol. 2, no. 4, August 1999 (1999-08), pages 211-221, XP001094530 ISSN: 1386-2073
- MASON JONATHAN S ET AL: "New 4-point pharmacophore method for molecular similarity and diversity applications: Overview of the method and applications, including a novel approach to the design of combinatorial libraries containing privileged substructures." JOURNAL OF MEDICINAL CHEMISTRY, vol. 42, no. 17, 26 August 1999 (1999-08-26), pages 3251-3264, XP002234658 ISSN: 0022-2623
- HAHN MATHEW: "Receptor Surface Models: 1. Definition and Construction." JOURNAL OF MEDICINAL CHEMISTRY, vol. 38, no. 12, 1995, pages 2080-2090, XP002234659 ISSN: 0022-2623
- OSHIRO C M ET AL: "Characterization of receptors with a new negative image: Use in molecular docking and lead optimization." PROTEINS, vol. 30, no. 3, 15 February 1998 (1998-02-15), pages 321-336, XP009007777 ISSN: 0887-3585
- SALO JUKKA-PEKKA ET AL: "Parameter refinement for molecular docking" J. CHEM. INF. COMPUT. SCI., vol. 38, 1998, pages 832-839, XP002234660
- PICKETT S D ET AL: "DIVERSITY PROFILING AND DESIGN USING 3D PHARMACOPHORES: PHARMACOPHORE-DERIVED QUERIES (PDQ)" JOURNAL OF CHEMICAL INFORMATION AND COMPUTER SCIENCES, AMERICAN CHEMICAL SOCIETY, COLOMBUS,OHIO, US, vol. 36, 1996, pages 1214-1223, XP002062096 ISSN: 0095-2338

## Description

The present invention relates to a method for generating a focussed compound library containing ligand compounds being capable of binding to a preselected target or receptor. In particular, the invention relates to an integrated lead identification approach starting out from the three-dimensional structure of a selected target or receptor.

One major bottleneck of current drug discovery and drug development is the search for lead structures. To find new lead structures great efforts are made on the development of fast screening tools. Currently combinatorial chemistry, high-throughput screening (HTS) and laborious medicinal chemistry are the major columns in drug discovery. These technologies result in an effective search of lead structures. Anyway this process is still very expensive and time-consuming. Therefore, computer-based algorithms will be significant improvement in minimizing costs and time.

It is therefore an objective of the present invention to provide a method for further improving and accelerating the virtual screening of databases for specific ligand compounds.

It is a further objective of the present invention to provide a method for generating a focussed compound library comprising ligand compounds being capable of binding to a preselected target.

It is another objective of the present invention to provide an improved method for generating pharmacophores.

The objectives of the present invention are solved by a method for generating a focussed compound library comprising the steps
(a) selecting a target,
(b) providing information about the three-dimensional structure of the target,
(c) identifying interaction points in the active site of the target from the three-dimensional structure,
(d) generating an inverse active site consisting of ligand atom positions expressed as interaction types, which describe the chemical properties of the ligand atoms and whereby the distance to the target atoms depends on the interaction type and whereas each target atom comprising a pharmacophoric property is examined, wherein the inverse active site is obtained by:
   i. generating a sphere around each target atom with a given radius depending on the interaction type;
   ii. filling the surface of the sphere with dots which obtain the inverse pharmacophoric property of the target atom,
(e) generating two or more shells of inverse active sites around each sphere generated around each target atom comprising pharmacophoric property and whereby those shells have different distances to the target, whereby the distances of the dots on each shell depend on the distance to the target atom,
(f) extracting a pharmacophore for a ligand by clustering interaction points of the inverse active sites of the shells by determining the density of dots on different spheres in space whereby regions with higher density are separated from regions with lower density,
(g) employing the inverse active site pharmacophore as query for the identification of suitable ligand compounds, and
(h) collecting the suitable ligand compounds identified so as to form a focussed library.

The method of the present invention, also called Surf2Lead® , is an integrated strategy for fast lead identification. The basic concept is the discovery of pharmaceutical lead compounds starting out from the three-dimensional target or receptor structure. In combination with computational screening approaches (e.g. PHACIR® ) new biologically active ligands can be found.

The present invention, called Surf2Lead® , relates to a novel lead detection strategy. Surf2Lead® identifies ligands capable of specifically binding given targets of protein domains based on the 3D information of the target.

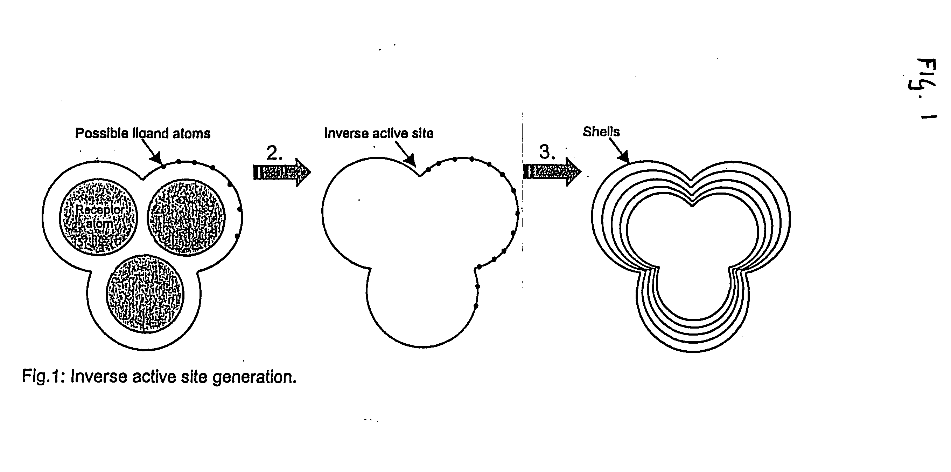

The Surf2Lead® strategy comprises the following steps:
1. Identification of possible interaction points in the active site of a given target. Any 3D target structure (X-ray, NMR, theoretical model) or any 3D active site information can be used.
2. Generation of an "inverse active site" to define possible ligand atom positions in the active site. Atoms are expressed as interaction types describing their chemical properties (Fig.1). The distance to the target atoms depends on the interaction type.
3. Several shells of inverse active sites with different distance to the target are generated for each interaction type. The density of possible ligand atoms on these shells depend on the distance to the target.
4. The shells are used to extract a possible pharmacophore describing possible ligands by clustering the interaction points. The clustering depends on a given radius around each dot which is examined and on the density of the dots on the shells. Optionally, the distance to the receptor can be taken into account. And optionally
5. Lead compound identification: The inverse active site pharmacophore serves as a query for computer-based lead identification tools (e.g. PHACIR® ). The resulting focussed library of potential leads has to be tested in vitro.

According to the method of the invention, first, a desired target is selected, e.g. a target associated with a particular disease. Information about suitable targets can be obtained, for example, by previous testing or from literature.

Next, the three-dimensional structure of the target is determined according to the invention. Thereby either the three-dimensional structure of the whole target or only the three-dimensional structure of parts can be determined. In particular, the three-dimensional information about active sites or the three-dimensional structure of target pockets is sufficient in many cases. The three-dimensional structure actually can be determined, e.g. by X-ray structure analysis, NMR or other methods, or estimated in a theoretical model.

Next, interaction points in the active site of the target are identified from the three-dimensional structure. Each target atom will be analysed according to their pharmacophoric properties (e.g. hydrogen bond acceptor, lipophilic, etc.). These properties are defined within a table describing different functional groups and their pharmacophoric pattern. The resulting pharmacophoric properties of the target atoms serve as the base for the concurrent generation of the inverse active site.

Next, an inverse active site is generated consisting of ligand atom positions expressed as interaction types. The interaction types describe the chemical properties of the ligand atoms, whereby the distance to the target atoms depends on the interaction type. Examples of interaction types are hydrogen bond donor, hydrogen bond acceptor, lipophilic, aromatic, positive or negative. To obtain an inverse active site, each target atom comprising a pharmacophoric property is examined. At first, a sphere is generated around this target atom with a given radius depending on the interaction type. This surface of this sphere is filled with dots. The dots obtain the "inverse" pharmacophoric property of the target atom, e.g. hydrogen bond acceptor target atom is surrounded by hydrogen bond donor dots. These dots represent potential ligand atom positions (PLAP). To examine only the surface dots all inner dots within the target structure will be deleted. By executing this procedure a surface shell for each interaction type can be obtained.

In the method of the invention two or more shells of inverse active sites having different distances to the target are generated for each interaction type. More preferably, 3 to 20, in particular, 4 to 10 shells are formed. The radii of the shells normally range within empirically determined values for the distinct interaction types, e.g. lipophilic from 2.5 to 4.5Å, but can vary in a wide range. The distances of the dots on each shell depend on the distance to the target atom and will be higher for the outer and inner shells and smaller for middle shells to attain a stronger weight of the medium distances of interactions. The dot distance can vary in a wide range of 0.01 up to 10Å.

Finally, a pharmacophore is extracted by clustering the interaction points of the active inverse sites of the shells. The obtained PLAP shells serve as the base for extracting pharmacophoric information by means of clustering. This is effected by determining the density of the PLAPs in space. Regions with higher density are separated from regions with lower density. From each cluster pivotal points are selected, representing pharmacophoric points. The number of representative pharmacophoric points is flexible and depends on the preset density and radii.

Providing a pharmacophore as described above is one aspect of the invention.

The thus obtained pharmacophore serves as query, e.g. for computer-based lead identification tools for the identification of suitable ligand or lead compounds. In particular, small molecules having a molecular weight of between 100 and 2,000 Dalton, in particular, between 150 and 1,000 Dalton, which are no peptides, are preferred lead structures.

The identified lead compounds can be collected or combined in a library. It is also possible to generate a focussed library of potential lead candidates by means of a limit value, starting out from a starting compound library. The lead candidates can then be tested for their activity in vitro.

The invention particularly includes the enrichment of biologically active compounds in a focussed substance library which is obtained from given starting databases. The pharmacophores generated according to the invention can be used for the extraction of ligand compounds from a substance library or for focussing a database according to specific similarity criteria.

The method according to the invention, in particular, can be employed for the development of lead structures, drugs or biologically active compounds. It is particularly suitable for applications in human or veterinary medicine and in plant protection.

The invention is further described by the following Examples and the Figures, wherein
- Fig.1: shows an inverse active site generation

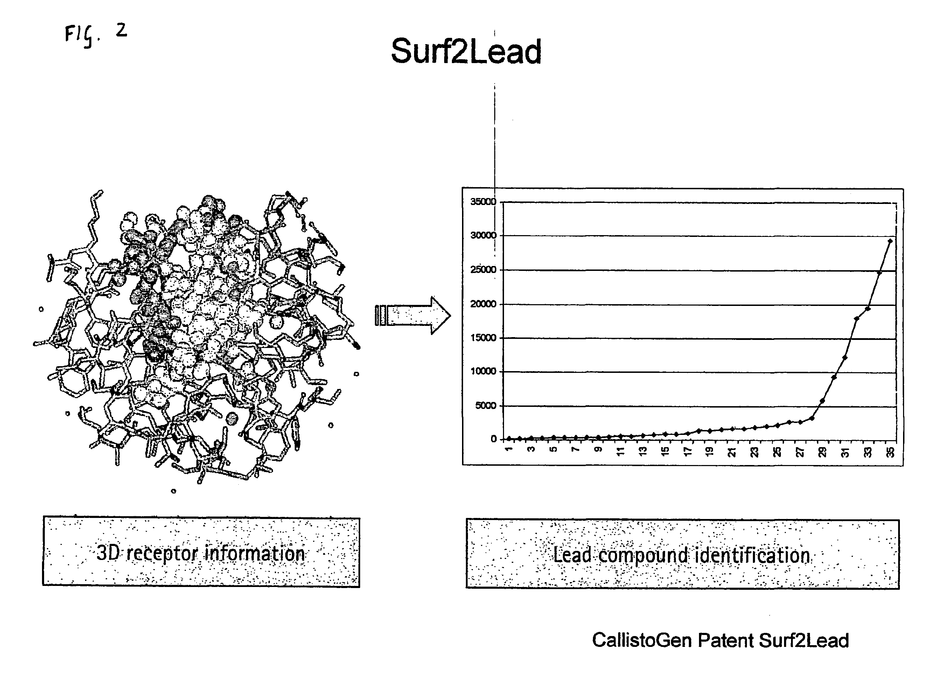

- Fig.2: shows the concept of lead compound identification starting out from 3D target information, and

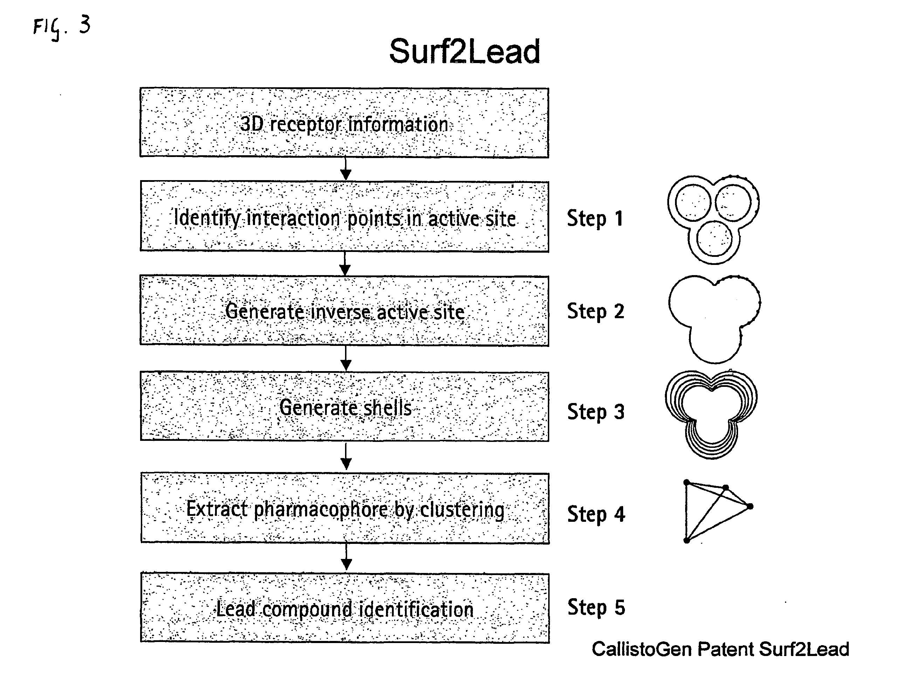

- Fig.3: shows an overview of the Surf2Lead® integrated lead identification approach.

## Claims

1. A method for generating a focussed compound library comprising the steps
(a) selecting a target,
(b) providing information about the three-dimensional structure of the target,
(c) identifying interaction points in the active site of the target from the three-dimensional structure,
(d) generating an inverse active site consisting of ligand atom positions expressed as interaction types, which describe the chemical properties of the ligand atoms and whereby the distance to the target atoms depends on the interaction type and whereas each target atom comprising a pharmacophoric property is examined, wherein the inverse active site is obtained by:
i. generating a sphere around each target atom with a given radius depending on the interaction type;
ii. filling the surface of the sphere with dots which obtain the inverse pharmacophoric property of the target atom,
(e) generating two or more shells of inverse active sites around each target atom comprising pharmacophoric property and whereby those shells have different distances to the target, whereby the distances of the dots on each shell depend on the distance to the target atom,
(f) extracting a pharmacophore for a ligand by clustering interaction points of the inverse active sites of the shells by determining the density of dots on different spheres in space whereby regions with higher density are separated from regions with lower density,
(g) employing the inverse active site pharmacophore as query for the identification of suitable ligand compounds, and
(h) collecting the suitable ligand compounds so as to form a focused library.

2. A method for generating a pharmacophore comprising the steps:
(a) selecting a target,
(b) providing information about the three-dimensional structure of the target,
(c) identifying interaction points in the active site of the target from the three-dimensional structure,
(d) generating an inverse active site consisting of ligand atom positions expressed as interaction types, which describe the chemical properties of the ligand atoms and whereby the distance to the target atoms depends on the interaction type and whereas each target atom comprising a pharmacophoric property is examined, wherein the inverse active site is obtained by:
i. generating a sphere around each target atom with a given radius depending on the interactive type;
ii. filling the surface of the sphere with dots which obtain the inverse pharmacophoric property of the target atom,
(e) generating two or more shells of inverse active around each target atom comprising pharmacophoric property and whereby those shells have different distances to the target, whereby the distances of the dots on each shell depend on the distance to the target atom,
(f) extracting a pharmacophore for a ligand by clustering interaction points of the inverse active sites of the shells by determining the density of dots on different spheres in space whereby regions with higher density are separated from regions with lower density.

3. The method according to claim 1 or 2,
wherein a two-point pharmacophore, a three-point pharmacophore or a four-point pharmacophore is generated.

4. The method according to any of the preceding claims,
wherein a target is selected which is associated with a particular disease.

5. The method according to any of the preceding claims,
wherein the three-dimensional structure of the whole target or the three-dimensional structure of parts of the target is provided.

6. The method according to claim 5,
wherein the three-dimensional structure is obtained by a theoretical model or/and experiments.

7. The method according to any of the preceding claims,
wherein interaction points are identified by assigning pharmacophoric properties to the target atoms.

8. The method according to any of the preceding claims,
wherein an inverse active site is generated by spheres around interaction points covered with dots representing potential ligand atom positions.

9. The method according to any of the preceding claims,
wherein 3 to 20 shells of inverse active sites are generated.

## Patentansprüche

1. Verfahren zum Erzeugen einer Fokusverbindung-Bibliothek, umfassend die Schritte
(a) Auswählen eines Targets,
(b) Bereitstellen von Information über die dreidimensionale Struktur des Targets,
(c) Identifizieren von Wechselwirkungspunkten in der aktiven Stelle des Targets aus der dreidimensionalen Struktur,
(d) Erzeugen einer inversen aktiven Stelle, bestehend aus Ligandenatompositionen, ausgedrückt als Wechselwirkungstypen, welche die chemischen Eigenschaften der Ligandenatome beschreiben und wobei der Abstand der Targetatome von dem Wechselwirkungstyp abhängt und wobei jedes Targetatom, das eine pharmacophore Eigenschaft aufweist, untersucht wird, worin die inverse aktive Stelle erhalten wird durch:
i. Erzeugen einer Sphäre um jedes Targetatom mit einem gegebenen Radius in Abhängigkeit vom Wechselwirkungstyp;
ii. Füllen der Oberfläche der Sphäre mit Punkten, die die inverse pharmacophore Eigenschaft des Targetatoms erhalten,
(e) Erzeugen von zwei oder mehreren Hüllen inverser aktiver Stellen um jedes Targetatom, umfassend pharmacophore Eigenschaft, und wobei diese Hüllen verschiedene Abstände zum Target aufweisen, wobei die Abstände der Punkte auf jeder Hülle von dem Abstand zum Targetatom abhängen,
(f) Extrahieren eines Pharmacophors für einen Liganden durch Clustering von Wechselwirkungspunkten inverser aktiver Stellen der Hüllen durch Bestimmen der Dichte von Punkten auf verschiedenen Sphären im Raum, wobei Regionen mit höherer Dichte von Regionen mit niedrigerer Dichte getrennt werden,
(g) Verwenden des Pharmacophors mit inverser aktiver Stelle als Suchanfrage zur Identifikation geeigneter Ligandenverbindungen und
(h) Sammeln der geeigneten Ligandenverbindungen, um eine Fokusbibliothek zu bilden.

2. Verfahren zum Erzeugen eines Pharmacophors, umfassend die Schritte:
(a) Auswählen eines Targets,
(b) Bereitstellen von Information über die dreidimensionale Struktur des Targets,
(c) Identifizieren von Wechselwirkungspunkten in der aktiven Stelle des Targets aus der dreidimensionalen Struktur,
(d) Erzeugen einer inversen aktiven Stelle, bestehend aus Ligandenatompositionen, ausgedrückt als Wechselwirkungstypen, welche die chemischen Eigenschaften der Ligandenatome beschreiben, und wobei der Abstand zu den Targetatomen abhängt von dem Wechselwirkungstyp und wobei jedes Targetatom, das pharmacophore Eigenschaften aufweist, untersucht wird, worin die inverse aktive Stelle erhalten wird durch:
i. Erzeugen einer Sphäre um jedes Targetatom mit einem gegebenen Radius, der von dem Wechselwirkungstyp abhängt;
ii. Füllen der Oberfläche der Sphäre mit Punkten, welche die inverse pharmacophore Eigenschaft des Targetatoms enthalten,
(e) Erzeugen von zwei oder mehr Hüllen inverser aktiver Stellen um jedes Targetatom, umfassend pharmacophore Eigenschaft, und worin diese Hüllen verschiedene Abstände zum Target aufweisen, wobei die Abstände der Punkte auf jeder Hülle von dem Abstand zum Targetatom abhängen,
(f) Extrahieren eines Pharmacophors für einen Liganden durch Clustering von Wechselwirkungspunkten der inversen aktiven Stellen der Hüllen durch Bestimmen der Dichte der Punkte auf verschiedenen Sphären im Raum, wobei Regionen mit höherer Dichte von Regionen mit geringerer Dichte getrennt werden.

3. Verfahren nach Anspruch 1 oder 2, worin ein Zweipunkt-Pharmacophor, ein Dreipunkt-Pharmacophor oder ein Vierpunkt-Pharmacophor erzeugt wird.

4. Verfahren nach einem der vorhergehenden Ansprüche, worin ein Target ausgewählt wird, das mit einer speziellen Krankheit assoziiert ist.

5. Verfahren nach einem der vorhergehenden Ansprüche, worin die dreidimensionale Struktur des Gesamttargets oder die dreidimensionale Struktur von Teilen des Targets bereitgestellt wird.

6. Verfahren nach Anspruch 5, worin die dreidimensionale Struktur erhalten wird durch ein theoretisches Modell und/oder Experimente.

7. Verfahren nach einem der vorhergehenden Ansprüche, worin Wechselwirkungspunkte identifiziert werden indem pharmacophore Eigenschaften den Targetatomen zugeschrieben werden.

8. Verfahren nach einem der vorhergehenden Ansprüche, worin eine inverse aktive Stelle erzeugt wird durch Sphären um Wechselwirkungspunkte, die mit Punkten überzogen sind, die potenzielle Ligandenatompositionen darstellen.

9. Verfahren nach einem der vorhergehenden Ansprüche, worin 3 bis 20 Hüllen von inversen aktiven Stellen erzeugt werden.

## Revendications

1. Procédé pour générer une bibliothèque de composés focalisés comprenant les étapes consistant à
(a) choisir une cible,
(b) fournir des informations sur la structure tridimensionnelle de la cible,
(c) identifier les points d'interaction dans le site actif de la cible à partir de la structure tridimensionnelle,
(d) générer un site actif inverse consistant en positions d'atomes de ligands exprimés en tant que types d'interaction, qui décrivent les propriétés chimiques des atomes de ligands, et la distance jusqu'aux atomes cibles étant fonction du type d'interaction et tandis que l'on examine chaque atome cible comprenant une propriété pharmacophore, procédé dans lequel le site actif inverse est obtenu par :
i. la génération d'une sphère autour de chaque atome cible avec un rayon donné dépendant du type d'interactiori ;
ii. le remplissage de la surface de la sphère avec des points qui obtiennent la propriété pharmacophore inverse de l'atome cible,
(e) générer deux ou plus de deux coques de sites actifs inverses autour de chaque atome cible comprenant la propriété pharmacophore et ces coques ayant des distances différentes jusqu'à la cible, les distances des points sur chaque coque étant fonction de la distance jusqu'à l'atome cible,
(f) extraire un pharmacophore pour un ligand par regroupement des points d'interaction des sites actifs inverses des coques en déterminant la densité des points sur différentes sphères dans l'espace, les régions de plus forte densité étant séparées des régions de plus faible densité,
(g) employer le pharmacophore de site actif inverse en tant qu'interrogation pour l'identification des composés de ligands appropriés, et
(h) recueillir les composés de ligands appropriés, de façon à former une bibliothèque focalisée.

2. Procédé pour générer un pharmacophore comprenant les étapes consistant à :
(a) choisir une cible,
(b) fournir des informations sur la structure tridimensionnelle de la cible,
(c) identifier les points d'interaction dans le site actif de la cible à partir de la structure tridimensionnelle,
(d) générer un site actif inverse consistant en positions d'atomes de ligands exprimés en tant que types d'interaction, qui décrivent les propriétés chimiques des atomes de ligands, et la distance jusqu'aux atomes cibles étant fonction du type d'interaction et tandis que l'on examine chaque atome cible comprenant une propriété pharmacophore, procédé dans lequel le site actif inverse est obtenu par :
i. génération d'une sphère autour de chaque atome cible avec un rayon donné dépendant du type interactif ;
ii. remplissage de la surface de la sphère avec des points qui obtiennent la propriété pharmacophore inverse de l'atome cible,
(e) générer deux ou plus de deux coques de sites actifs inverses autour de chaque atome cible comprenant la propriété pharmacophore et ces coques ayant des distances différentes jusqu'à la cible, les distances des points sur chaque coque étant fonction de la distance jusqu'à l'atome cible,
(f) extraire un pharmacophore pour un ligand par regroupement des points d'interaction des sites actifs inverses des coques en déterminant la densité des points sur différentes sphères dans l'espace, les régions de plus forte densité étant séparées des régions de plus faible densité.

3. Procédé selon la revendication 1 ou 2, dans lequel il est généré un pharmacophore à deux points, un pharmacophore à trois points ou un pharmacophore à quatre points.

4. Procédé selon l'une quelconque des revendications précédentes, dans lequel on choisit une cible qui est associée à une maladie particulière.

5. Procédé selon 1'une quelconque des revendications précédentes, dans lequel est fournie la structure tridimensionnelle de la cible complète ou la structure tridimensionnelle des parties de la cible.

6. Procédé selon la revendication 5, dans lequel la structure tridimensionnelle est obtenue par un modèle théorique et/ou des expériences.

7. Procédé selon l'une quelconque des revendications précédentes, dans lequel les points d'interaction sont identifiés par affectation des propriétés pharmacophores aux atomes cibles.

8. Procédé selon l'une quelconque des revendications précédentes, dans lequel un site actif inverse est généré par des sphères autour de lieux d'interaction couverts de points représentant des positions d'atomes de ligands potentiels.

9. Procédé selon l'une quelconque des revendications précédentes, dans lequel sont générées 3 à 20 coques de sites actifs inverses.
